(19) Europäisches Patentamt
European Patent Office
Office européen des brevets

(11) EP 4 276 087 A1

(12) **EUROPEAN PATENT APPLICATION**
published in accordance with Art. 153(4) EPC

(43) Date of publication:
**15.11.2023 Bulletin 2023/46**

(21) Application number: **22924566.7**

(22) Date of filing: **17.11.2022**

(51) International Patent Classification (IPC):
***C07C 43/215*** *(2006.01)* ***C07C 41/30*** *(2006.01)*
***C07C 69/767*** *(2006.01)* ***C07B 37/00*** *(2006.01)*
***A61K 31/085*** *(2006.01)* ***A61K 31/277*** *(2006.01)*

(52) Cooperative Patent Classification (CPC):
Y02P 20/55

(86) International application number:
**PCT/CN2022/132420**

(87) International publication number:
**WO 2023/160035 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.02.2022 CN 202210185780**

(71) Applicant: **Hangzhou Normal University**
**Hangzhou, Zhejiang 311121 (CN)**

(72) Inventors:
- **XIE, Tian**
  **Zhejiang 311121 (CN)**
- **YE, Yang**
  **Zhejiang 311121 (CN)**
- **XU, Bing**
  **Zhejiang 311121 (CN)**
- **YE, Xiangyang**
  **Zhejiang 311121 (CN)**

(74) Representative: **Alatis**
**3, rue Paul Escudier**
**75009 Paris (FR)**

# (54) BETA-ELEMENE VINYLATION COUPLED DERIVATIVE, AND PREPARATION THEREOF AND USE THEREOF IN PREPARATION OF ANTITUMOR DRUG

(57) The present disclosure provides a vinylation coupled derivative of β-elemene, and a preparation method and use thereof in preparation of an antitumor drug. The present disclosure provides a vinylation coupled derivative of β-elemene having a structure shown in Formula (I), a pharmaceutical composition and a hydrate including a compound shown in Formula (I), as well as an isotopic derivative, a chiral isomer, a variant, a salt, a prodrug, and a preparation of the compound shown in Formula (I). The present disclosure further provides a preparation method and use of the vinylation coupled derivative of β-elemene, and an inhibitory activity of the derivative on proliferation of various tumor cell lines. The vinylation coupled derivative of β-elemene is expected to be an antitumor drug candidate for treating colon cancer and lung cancer.

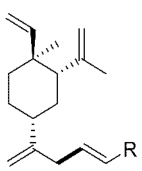

Formula (I)

EP 4 276 087 A1

## Description

### CROSS REFERENCE TO RELATED APPLICATION

The present disclosure claims priority to the Chinese Patent Application CN202210185780.6 filed to the China National Intellectual Property Administration (CNIPA) on February 28, 2022 and entitled "VINYLATION COUPLED DERIVATIVE OF BETA-ELEMENE, AND PREPARATION METHOD AND USE THEREOF IN PREPARATION OF ANTITUMOR DRUG", which is incorporated herein by reference in its entirety.

### TECHNICAL FIELD

The present disclosure relates to the technical field of preparation of β-elemene derivatives, in particular to a vinylation coupled derivative of β-elemene, and a preparation method and use thereof in preparation of an antitumor drug.

### BACKGROUND

Elemene as a type of small-molecule volatile oil compound is mainly extracted from the tuber of *Curcuma wenyujin.* Elemene reported in existing literatures mainly includes α-elemene, (±)-β-elemene, γ-elemene, and δ-elemene. The (-)-β-elemene is the main active ingredient exerting anti-tumor effects, has a broad-spectrum anti-tumor activity, and shows certain curative effects on various cancers, such as liver cancer, breast cancer, and lung cancer. At present, a variety of preparations with the elemene as a main component have achieved certain anticancer effects in clinical practice.

β-Elemene is one of the few new anti-tumor drugs with independent intellectual property rights in China. The research on the derivatives of β-elemene has made great progress in recent years. According to the research results of a structure-activity relationship, the double bond and the backbone of β-elemene have an important influence on its activity. The activities of β-elemene compounds without three double bonds being destroyed are not greatly affected. However, low polarity, poor water solubility, and low oral bioavailability limit the clinical applications of elemene. Therefore, it is necessary to modify the structure of elemene to obtain elemene derivatives with desirable water solubility, higher oral bioavailability, better biological activity than that of elemene, and less toxic and side effects.

The structural modification of β-elemene has been developed since the 1970s. There are more than 150 kinds of derivatives of β-elemene that have been synthesized, involving several major categories such as reduction and oxidation, sulfur-containing, nitrogen-containing, esters, ethers, alcohols, and glycosides. Currently, there are very few studies on the vinylation coupling of β-elemene.

### SUMMARY

A first purpose of the present disclosure is to address the deficiencies of the prior art, and to construct a series of vinylation coupled derivatives of β-elemene with β-elemene as a matrix. The present disclosure provides a vinylation coupled derivative of β-elemene, and researches its antitumor activity, so as to develop a new antitumor drug.

The present disclosure provides a vinylation coupled derivative of β-elemene, or an optical isomer, a racemate, a single enantiomer, and a possible diastereomer thereof, or a pharmaceutically acceptable salt, a prodrug, a deuterated derivative, a hydrate, and a solvate thereof, where the vinylation coupled derivative of β-elemene has a structure shown in formula (I):

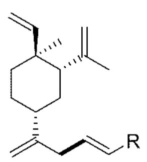

Formula (I);

and

in Formula (I):

R is any one independently selected from the group consisting of the following structural fragments:

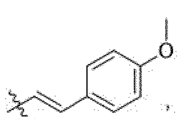, 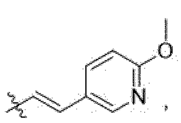, 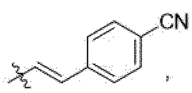, 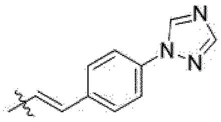,

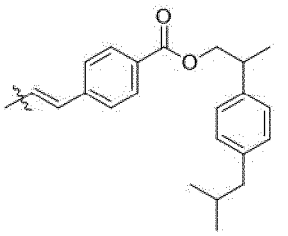, 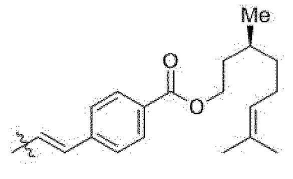, 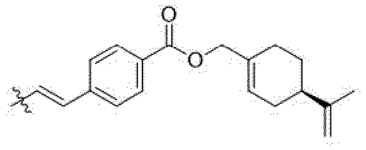,

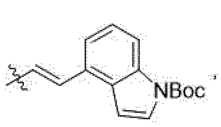, 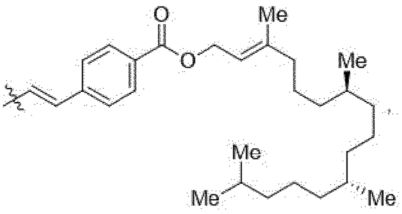, 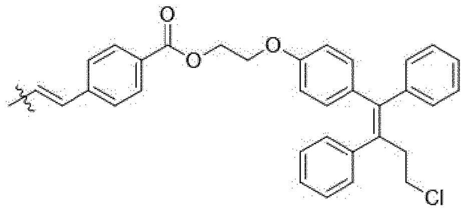,

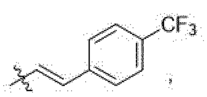, 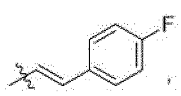, 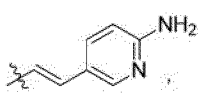, 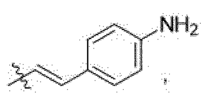,

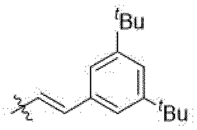, 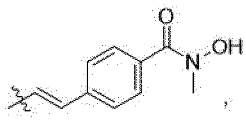, 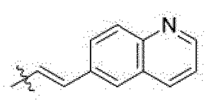, 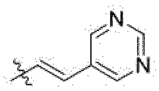,

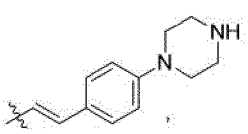, 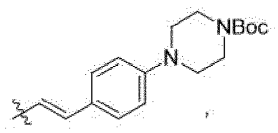, 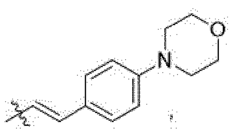, 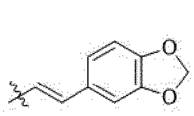.

Further, the vinylation coupled derivative of β-elemene has a structure shown in any one of the Compounds 1 to 22:

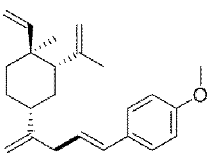

1

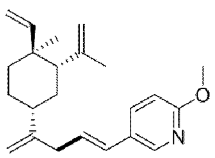

2

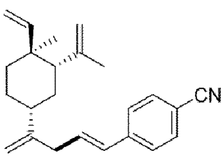

3

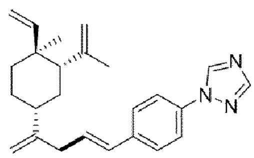

4

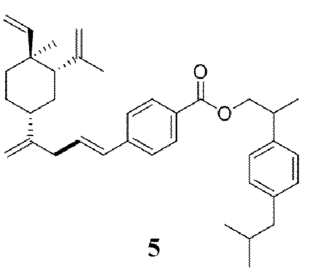

5

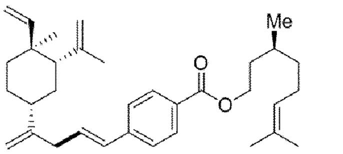

6

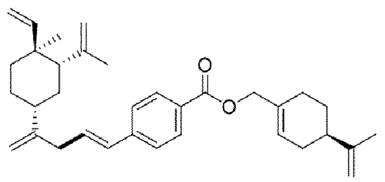

7

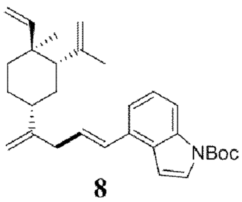

8

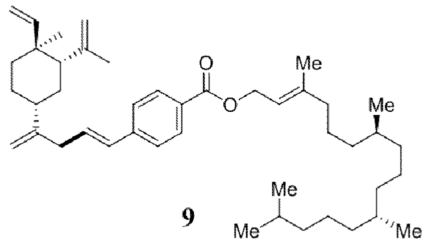

9

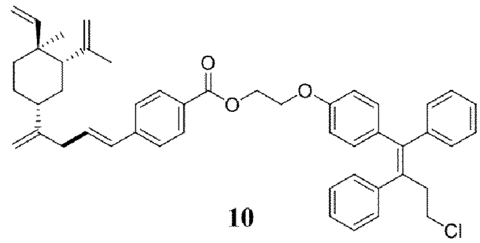

10

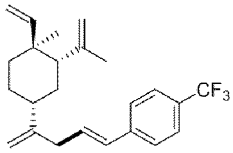

11

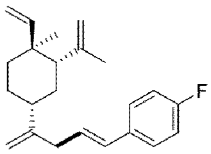

12

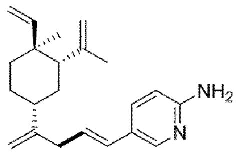

13

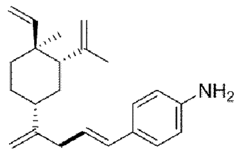

14

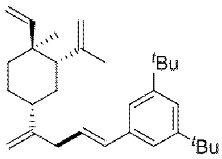

15

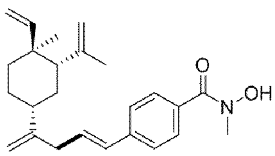

16

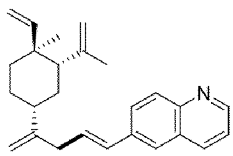

17

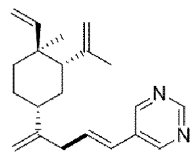

18

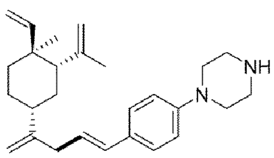

19

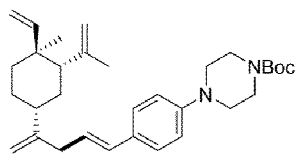

20

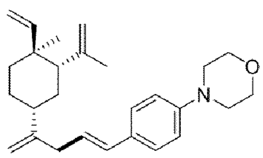

21

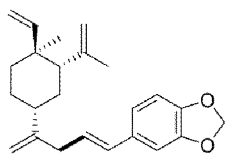

22

A second purpose of the present disclosure is to provide a preparation method of the vinylation coupled derivative of β-elemene.

In the present disclosure, the following synthetic route can be adopted for the vinylation coupled derivative of β-elemene having the structure shown in Formula (I):

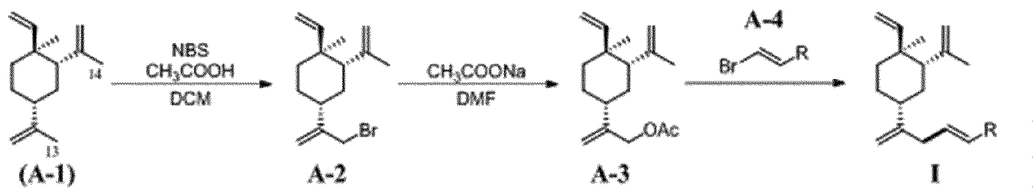


(A-1) A-2 A-3 I

β-elemene

R = aryl, heteroaryl, and alkyl

;

and

the preparation method includes the following steps:

(1) subjecting β-elemene A-1 to bromine substitution at an allyl position 13 to obtain an intermediate A-2;

(2) subjecting the intermediate A-2 to selective nucleophilic substitution to obtain an intermediate A-3; and

(3) allowing the intermediate A-3 and alkenyl bromide A-4 to have vinylation coupling to obtain the vinylation coupled derivative of β-elemene having a structure shown in Formula (I).

In the present disclosure, the vinylation coupled derivative of β-elemene having a structure shown in Formula (I) is prepared by the above method. However, the conditions of the above methods, such as reactants, solvents, amounts of compounds used, reaction temperature, time required for reaction, are not limited to the above explanations. The compound can also be conveniently prepared by optionally combining various synthetic methods described in this description or known in the art. Such combinations can be easily conducted by those skilled in the art to which the present disclosure pertains.

In the present disclosure, the step (1) of each synthetic route can adopt the prior art, as disclosed in the patent CN110683932A.

A third purpose of the present disclosure is to provide use of the vinylation coupled derivative of β-elemene, or an optical isomer, a racemate, a single enantiomer, and a possible diastereomer thereof, or a pharmaceutically acceptable salt, a prodrug, a deuterated derivative, a hydrate, and a solvate thereof in preparation of an antitumor drug.

A fourth purpose of the present disclosure is to provide an antitumor drug a safe and effective dosage of the vinylation coupled derivative of β-elemene, or an optical isomer, a racemate, a single enantiomer, and a possible diastereomer thereof, or a pharmaceutically acceptable salt, a prodrug, a deuterated derivative, a hydrate, and a solvate thereof.

Preferably, the antitumor drug may further include a pharmaceutically acceptable carrier.

Preferably, in the use and the antitumor drug, the tumor includes colon cancer and lung cancer.

In the present disclosure, the compound has the activity of inhibiting the proliferation of various tumor cell lines. Therefore, the compound and its various crystal forms, pharmaceutically acceptable inorganic or organic salts, hydrates or solvates, and pharmaceutical compositions including the compound of the present disclosure as a main active ingredient can be used for the treatment, prevention, and alleviation of various diseases, including various cancers.

In the present disclosure, the pharmaceutical composition includes the compound of the present disclosure or a pharmaceutically acceptable salt thereof and a pharmaceutically acceptable excipient or carrier within a safe and effective amount range. Herein, the "safe and effective dose" refers to: an amount of the compound is sufficient to improve the condition obviously without causing serious side effects. Generally, the pharmaceutical composition includes 1 mg to 2,000 mg of the compound of the present disclosure/dose, more preferably 5 mg to 1,000 mg of the compound of the present disclosure/dose. Preferably, the "one dose" refers to one capsule or one tablet.

The "pharmaceutically acceptable carrier" refers to: one or more compatible solid or liquid fillers or gel substances, which are suitable for human and must have sufficient purity and low enough toxicity. The "compatibility" here means that each component in the composition can be blended with the compound of the present disclosure and with each other without significantly reducing the efficacy of the compound. Some examples of the pharmaceutically acceptable carrier include: cellulose and derivatives thereof (such as sodium carboxymethylcellulose, sodium ethylcellulose, and cellulose acetate), gelatin, talc, solid lubricants (such as stearic acid and magnesium stearate), calcium sulfate, vegetable oils (such as soybean oil, sesame oil, peanut oil, and olive oil), polyols (such as propylene glycol, glycerin, mannitol, and sorbitol), emulsifiers, wetting agents (such as ten sodium lauryl sulfate), coloring agents, flavoring agents, stabilizers, antioxidants, preservatives, and pyrogen-free water.

In the present disclosure, there is no special limitation on an administration mode of the compound or the pharmaceutical composition, and representative administration modes include (but not limited to): oral administration, intratumoral administration, rectal administration, parenteral administration (intravenous, intramuscular, or subcutaneous administration), and topical administration.

A solid dosage form for the oral administration includes a capsule, a tablet, a pill, a powder, and a granule. In the solid dosage form, the active compound is mixed with at least one conventional inert excipient (or carrier), such as sodium citrate or dicalcium phosphate, or with the following ingredients: (a) fillers or solubilizers, such as starch, lactose, sucrose, glucose, mannitol, and silicic acid; (b) binders, such as hydroxymethylcellulose, alginate, gelatin, polyvinylpyrrolidone, sucrose, and arabic gum; (c) humectants, such as glycerin; (d) disintegrants, such as agar, calcium carbonate, potato or tapioca starch, alginic acid, certain complex silicate, and sodium carbonate; (e) retarding solvent, such as paraffin; (f) absorption accelerators, such as quaternary ammonium compounds; (g) wetting agents, such as cetyl alcohol and glyceryl monostearate; (h) adsorbents, such as kaolin; (i) lubricants, such as talc, calcium stearate, magnesium stearate, solid polyethylene glycol, and sodium lauryl sulfate, or mixtures thereof. In capsules, tablets, and pills, the dosage form may further include a buffer.

Solid dosage forms such as tablets, dragees, capsules, pills, and granules can be prepared with coatings and shell materials such as enteric coatings and others well known in the art. These dosage forms may contain an opacifying agent, and the active compound or release of the compound from such compositions may be in a delayed manner at a site within the alimentary canal. Examples of usable embedding components are polymeric substances and waxy substances. The active compound can also be in a microencapsulated form, if desired, with one or more of the above-mentioned excipients.

Liquid dosage forms for oral administration include pharmaceutically acceptable emulsions, solutions, suspensions, syrups, or tinctures. Liquid dosage forms may include, in addition to the active compound, inert diluents such as

water or other solvents, solubilizers and emulsifiers conventionally used in the art. For example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil, and sesame oil or a mixture of these substances can be used.

Besides such inert diluents, the composition may also include adjuvants, such as wetting agents, emulsifiers, and suspending, sweetening, flavoring, and perfuming agents.

Suspensions, in addition to the active compounds, may include suspending agents such as ethoxylated isooctadecanol, polyoxyethylene sorbitol, and sorbitan esters, microcrystalline cellulose, aluminum methoxide, and agar, or mixtures of these substances.

Compositions for parenteral injection may include physiologically acceptable sterile aqueous or anhydrous solutions, dispersions, suspensions, or emulsions as well as sterile powders for reconstitution into sterile injectable solutions or dispersions. Suitable aqueous and non-aqueous carriers, diluents, solvents, or excipients include water, ethanol, polyols, and suitable mixtures thereof.

Dosage forms for topical administration of the compound in the present disclosure include ointments, powders, patches, sprays, and inhalants. The active ingredient is mixed under sterile conditions with a physiologically acceptable carrier and any preservatives, buffers, or propellants which may be necessary.

In the present disclosure, the compound can be administered alone or in combination with other pharmaceutically acceptable compounds.

When using a pharmaceutical composition, a safe and effective dosage of the compound of the present disclosure is applied to a mammal (such as a human) in need of treatment. The dosage during administration is a pharmaceutically effective dosage. For a person weighing 60 kg, a daily dosage is generally 1 mg to 5,000 mg, preferably 5 mg to 2,000 mg. Of course, factors such as an administration route and a health status of the patient should also be considered for the specific dosage, and are within the skill of skilled physicians.

Compared with the prior art, the main advantages of the present disclosure include: the present disclosure provides a vinylation coupled derivative of β-elemene having a structure shown in Formula (I), pharmaceutical compositions and hydrates including the vinylation coupled derivative of β-elemene having a structure shown in Formula (I), as well as isotopic derivatives, chiral isomers, variants, different salts, prodrugs, and preparations of the compound. The present disclosure further provides a preparation method and use of the vinylation coupled derivative of β-elemene, and an inhibitory activity of the derivative on proliferation of various tumor cell lines. The C($sp^3$)-C($sp^2$) bond-coupled derivatives of the β-elemene are expected to become anti-tumor drug candidates for the treatment of various cancers, such as lung cancer, liver cancer, colorectal cancer, gastric cancer, prostate cancer, ovarian cancer, breast cancer, or glioma.

## DETAILED DESCRIPTION OF THE EMBODIMENTS

The present disclosure will be further described below with reference to specific examples. It should be understood that these embodiments are only intended to describe the present disclosure, rather than to limit the scope of the present disclosure. In the following examples, the experimental methods in which specific conditions are not stated are generally conducted according to conventional conditions or according to the conditions recommended by the manufacturer.

### Example 1: Preparation of Compound 1

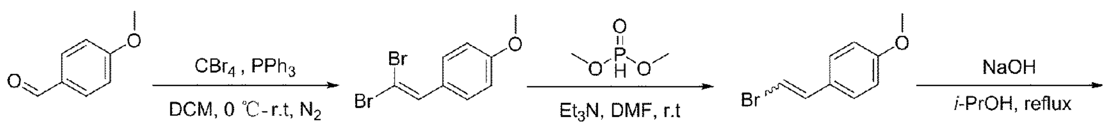


1a 1b 1c

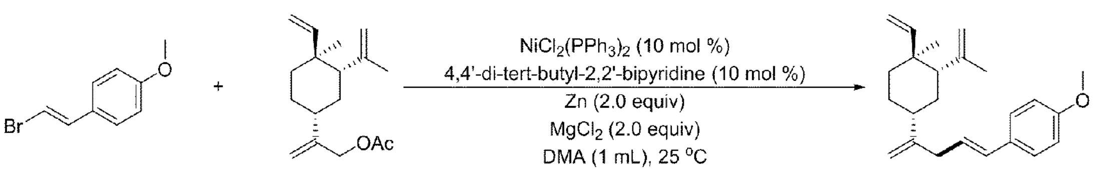


1d 1e 1

Intermediate **1d**

(1) $CBr_4$ (30 mmol, 150 mol %) was added to a dichloromethane (DCM, 80 mL) solution of Compound **1a** (20 mmol, 100 mol %) at 0°C; under nitrogen protection, a DCM (70 mL) solution of $PPh_3$ (60 mmol, 300 mol %) was added dropwise through a constant-pressure dropping funnel into a resulting mixture. A reaction was monitored by TLC. After the reaction was completed, about half of the volume of DCM was removed under reduced pressure, and then petroleum ether (PE, 100 mL) was added to precipitate triphenylphosphine oxide (TPPO). After filtration by diatomaceous earth and evaporation, an obtained residue was dissolved in PE (50 mL) to further precipitate the TPPO. The above steps were repeated several times to obtain a crude dibromide **1b** (-20 mmol, 100 mol %), which was directly used in the next step.

(2) An anhydrous N,N-dimethylformamide (DMF, 20 mL) solution of the crude dibromide **1b** (-20 mmol, 100 mol %) and NEts (60 mmol, 300 mol %) was added with dimethyl phosphite (60 mmol, 300 mol %) and stirred overnight under room temperature. DMF was distilled off under reduced pressure, an aqueous solution (60 mL) was added, a resulting product was extracted with PE (2×100 mL), an obtained combined organic phase was washed with an aqueous hydrochloric acid solution (1 M, 55 mL), and dried over anhydrous sodium sulfate. The above desiccant was removed by filtration, and a resulting filtrate was concentrated under reduced pressure to obtain a crude product **1c,** which was directly used in the next step.

(3) The crude product **1c** (-20 mmol, 100 mol %) was dissolved in isopropanol (i-PrOH, 30 mL). The solid sodium hydroxide (17 mmol, 85 mol %) was added to conduct reflux by heating for more than 5 h. A resulting reaction mixture was cooled to room temperature, extracted with *n*-hexane (100 mL), and an obtained combined organic phase was washed successively with an aqueous hydrochloric acid solution (1 M, 75 mL) and an aqueous solution (2×100 mL), and then dried over anhydrous sodium sulfate. The above desiccant was removed by filtration, an obtained filtrate was concentrated under reduced pressure, and an obtained crude product was purified by silica gel column chromatography (eluted with an ethyl acetate/petroleum ether (0.5%) system) to obtain a white solid **1d** (2.65 g, yield 62 %). $^1H$ NMR (500 MHz, $CDCl_3$) $\delta$ 7.25-7.21 (m, 2H), 7.04 (d, *J* = 14.0 Hz, 1H), 6.87-6.83 (m, 2H), 6.61 (d, *J* = 13.9 Hz, 1H), 3.81 (s, 3H).

Compound **1**

The intermediate **1d** (0.150 mmol, 1.0 equiv), intermediate **1e** (0.300 mmol, 2.0 equiv), a catalyst $NiCl_2(PPh_3)_2$ (9.8 mg, 0.015 mmol, 10 mol %), a reducing agent Zn powder (19.6 mg, 0.300 mmol, 2.0 equiv), a ligand 4,4'-di-tert-butyl-2,2'-bipyridine (4.0 mg, 0.015 mmol, 10 mol %), and an additive $MgCl_2$ (28.6 mg, 0.300 mmol, 2.0 equiv ) were added sequentially to an oven-dried Schlenk tube equipped with a magnetic stirrer. The Schlenk tube was evacuated three times through a double-row tube to ensure that the reaction system was under a nitrogen atmosphere, and finally an N,N-dimethylacetamide (DMA, 1.0 mL) solution was added with a syringe and stirred at 25°C for 12 h. After the reaction was completed, an obtained reaction mixture was directly purified by silica gel column chromatography (eluted with an ethyl acetate/petroleum ether (2%) system) without post-treatment to obtain a colorless oily liquid 1 (43.3 mg, yield 86%). $^1H$ NMR (500 MHz, $CDCl_3$) $\delta$ 7.31 (d, *J* = 8.7 Hz, 2H), 6.85 (d, *J* = 8.7 Hz, 2H), 6.37 (d, *J* = 15.8 Hz, 1H), 6.08 (dt, *J* = 15.7, 7.1 Hz, 1H), 5.83 (dd, *J* = 17.4, 10.9 Hz, 1H), 4.92 (dd, *J* = 8.1, 1.2 Hz, 1H), 4.89 (s, 1H), 4.87 (s, 1H), 4.83 (dd, *J* = 5.0, 1.3 Hz, 2H), 4.61 (s, 1H), 3.81 (s, 3H), 2.95 (d, *J* = 7.0 Hz, 2H), 2.02 (dd, *J* = 11.8, 4.3 Hz, 2H), 1.72 (s, 3H), 1.64-1.59 (m, 2H), 1.54-1.42 (m, 4H), 1.02 (s, 3H). $^{13}C$ NMR (126 MHz, $CDCl_3$) $\delta$ 158.92, 153.40, 150.38, 147.81, 130.81, 130.65, 127.27, 126.63, 114.06, 112.26, 110.01, 108.76, 55.41, 52.90, 44.30, 40.09, 39.98, 38.83, 33.36, 27.33, 24.93, 16.76. HRMS (ESI) m/z ($[M-H]^-$) calcd for $C_{24}H_{31}O$: 335.2380. Found: 335.2380.

**Example 2: Preparation of Compound 2**

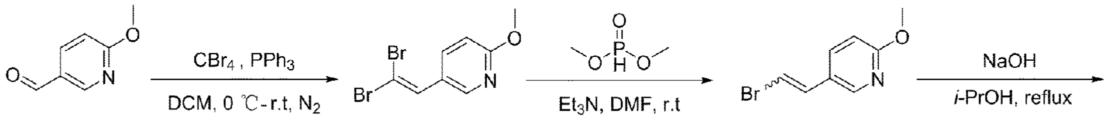


2a 2b 2c

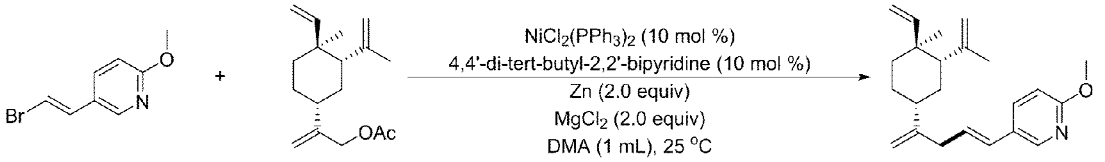


2d 1e 2

Intermediate **2d**

Referring to the synthesis steps of the intermediate **1d** in Example 1, a colorless oily liquid **2d** (881 mg, yield 21%) was obtained. $^1$H NMR (500 MHz, $CDCl_3$) $\delta$ 8.07 (d, *J* = 2.5 Hz, 1H), 7.55 (dd, *J* = 8.6, 2.5 Hz, 1H), 7.03 (d, *J* = 14.0 Hz, 1H), 6.71 (d, *J* = 8.7 Hz, 1H), 6.66 (d, *J* = 14.0 Hz, 1H), 3.94 (s, 3H).

Compound **2**

Referring to the synthesis steps of the Compound **1** in Example 1, a colorless oily liquid 2 (36.9 mg, yield 73%) was obtained. $^1$H NMR (500 MHz, $CDCl_3$) $\delta$ 8.08 (t, *J* = 5.5 Hz, 1H), 7.64 (dd, *J* = 8.6, 2.4 Hz, 1H), 6.69 (d, *J* = 8.6 Hz, 1H), 6.34 (d, *J* = 15.9 Hz, 1H), 6.10 (dt, *J* = 15.8, 7.0 Hz, 1H), 5.81 (dd, *J* = 17.3, 11.0 Hz, 1H), 4.99-4.75 (m, 5H), 4.59 (s, 1H), 3.93 (s, 3H), 2.95 (d, *J* = 6.9 Hz, 2H), 1.99 (ddd, *J* = 15.9, 11.2, 5.0 Hz, 2H), 1.71 (s, 3H), 1.65-1.56 (m, 3H), 1.50-1.41 (m, 3H), 1.01 (s, 3H). $^{13}$C NMR (126 MHz, $CDCl_3$) $\delta$ 163.43, 153.06, 150.32, 147.77, 145.08, 135.54, 128.25, 127.52, 126.97, 112.29, 110.93, 110.07, 109.01, 53.61, 52.90, 44.41, 40.02 (d, *J* = 11.8 Hz), 38.83, 33.37, 29.84, 27.32, 24.93, 16.76. HRMS (ESI) m/z ($[M+H]^+$) calcd for $C_{23}H_{32}NO$: 338.2478. Found: 338.2475.

**Example 3: Preparation of Compound 3**

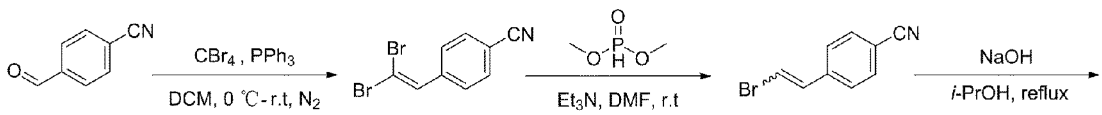


3a 3b 3c

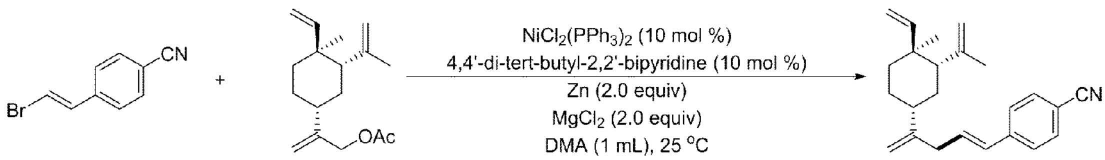


3d 1e 3

Compound **3**

Referring to the synthesis steps of the Compound 1 in Example 1, a colorless oily liquid **3** (39.7 mg, yield 80%) was obtained. $^1$H NMR (500 MHz, $CDCl_3$) $\delta$ 7.75 (d, *J* = 8.3 Hz, 2H), 7.43 (d, *J* = 8.3 Hz, 2H), 6.36 (ddd, *J* = 30.6, 15.8, 11.4 Hz, 2H), 5.81 (dd, *J* = 17.3, 11.0 Hz, 1H), 4.94-4.87 (m, 3H), 4.82 (d, *J*= 5.9 Hz, 2H), 4.59 (s, 1H), 2.99 (d, *J* = 6.8 Hz, 2H), 2.01 (dt, *J* = 18.0, 10.4 Hz, 2H), 1.71 (s, 3H), 1.64-1.56 (m, 3H), 1.52-1.42 (m, 3H), 1.01 (s, 3H). $^{13}$C NMR (126 MHz, $CDCl_3$) $\delta$ 169.12, 152.70, 150.29, 147.77, 141.53, 131.61, 130.50, 127.86, 126.29, 112.31, 110.10, 109.32, 100.12, 52.89, 44.48, 40.01 (d, *J* = 9.9 Hz), 38.82, 33.36, 29.84, 27.30, 24.93, 16.77. HRMS (ESI) m/z ($[M+H]^+$) calcd for $C_{24}H_{30}N$:

332.2373. Found: 332.2378.

**Example 4: Preparation of Compound 4**

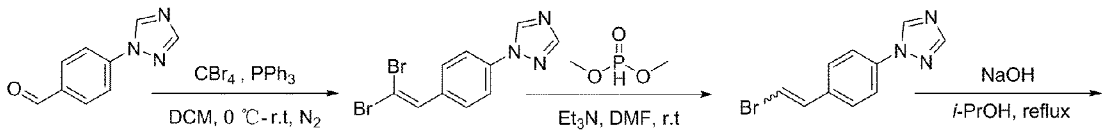


4a 4b 4c

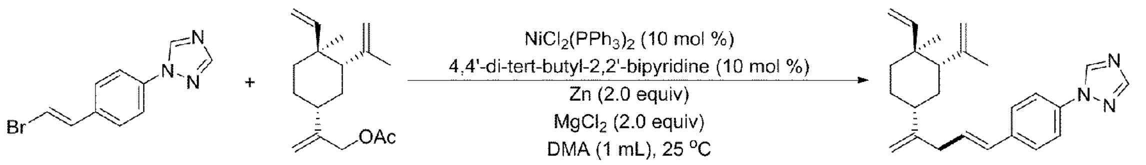


4d 1e 4

Compound **4**

Referring to the synthesis steps of the Compound 1 in Example 1, a colorless oily liquid **4** (43.1 mg, yield 77%) was obtained. $^1$H NMR (500 MHz, $CDCl_3$) $\delta$ 8.54 (s, 1H), 8.10 (s, 1H), 7.61 (d, *J* = 8.5 Hz, 2H), 7.49 (d, *J* = 8.6 Hz, 2H), 6.44 (d, *J* = 15.8 Hz, 1H), 6.29 (dt, *J* = 15.8, 7.0 Hz, 1H), 5.82 (dd, *J* = 17.3, 11.0 Hz, 1H), 4.97-4.87 (m, 3H), 4.83 (d, *J* = 1.4 Hz, 2H), 4.60 (s, 1H), 2.99 (d, *J*= 6.9 Hz, 2H), 2.06-1.97 (m, 2H), 1.71 (s, 3H), 1.64-1.58 (m, 3H), 1.52-1.42 (m, 3H), 1.01 (s, 3H). $^{13}$C NMR (126 MHz, $CDCl_3$) 152.81, 152.68, 150.29, 147.77, 140.84, 137.95, 135.73, 130.55, 130.06, 127.38, 120.29, 112.32, 110.11, 109.26, 52.90, 44.49, 40.06, 39.98, 38.78, 33.37, 27.32, 24.94, 16.77. HRMS (ESI) m/z ($[M+H]^+$) calcd for $C_{25}H_{32}N_3$: 374.2591. Found: 374.2590.

**Example 5: Preparation of Compound 5**

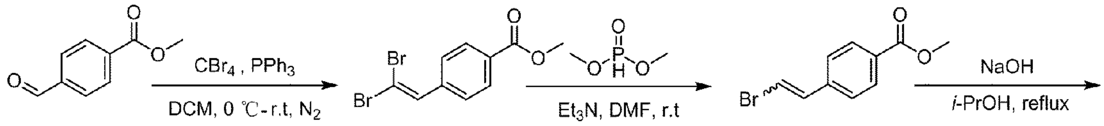


5a 5b 5c

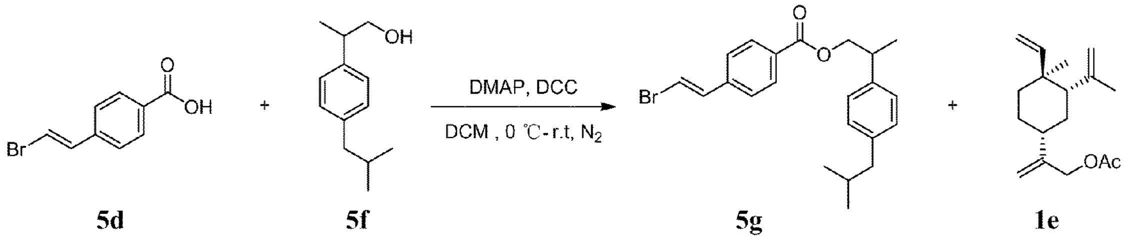


5d 5f 5g 1e

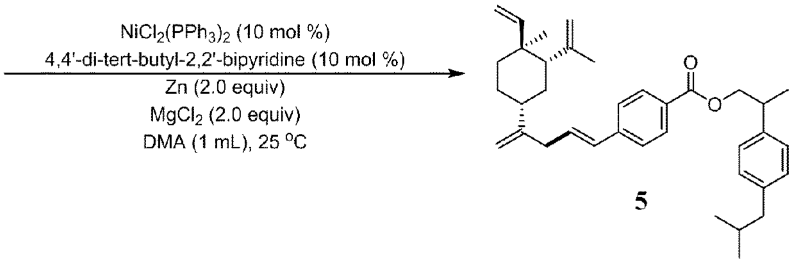


5

Intermediate **5g**

Referring to the synthesis steps of intermediate **1d** in Example 1, a white solid **5d** was obtained. In an ice bath, DMAP (37 mg, 0.3 mmol) and DCC (464 mg, 2.25 mmol) were sequentially added to an anhydrous DCM (10 mL) solution of the Compound **5d** (358 mg, 1.58 mmol) and Compound **5f** (288.4 mg, 1.5 mmol), and stirred overnight. DCM was distilled off under reduced pressure, and an aqueous solution (40 mL) was added to a resulting residue, followed by extraction with ethyl acetate (3×20 mL). A resulting combined organic phase was washed with saturated brine (2×20 mL), and then dried over anhydrous sodium sulfate. The above desiccant was removed by filtration, an obtained filtrate was concentrated under reduced pressure, and an obtained crude product was purified by silica gel column chromatography (eluted with an ethyl acetate/petroleum ether (0.5%) system) to obtain a white solid 5g (581 mg, yield 97%). $^1$H NMR (500 MHz, $CDCl_3$) $\delta$ 7.93 (d, *J* = 8.4 Hz, 2H), 7.32 (d, *J* = 8.3 Hz, 2H), 7.18 (d, *J* = 8.0 Hz, 2H), 7.15-7.07 (m, 3H), 6.90 (d, *J* = 14.0 Hz, 1H), 4.42-4.33 (m, 2H), 3.26-3.18 (m, 1H), 2.45 (d, *J* = 7.2 Hz, 2H), 1.84 (dt, *J* = 13.5, 6.8 Hz, 1H), 1.38 (d, *J* = 7.0 Hz, 3H), 0.89 (d, *J* = 6.6 Hz, 6H).

Compound **5**

Referring to the synthesis steps of the Compound 1 in Example 1, a colorless oily liquid 5 (67.6 mg, yield 86%) was obtained. $^1$H NMR (500 MHz, $CDCl_3$) $\delta$ 7.91 (d, *J* = 7.9 Hz, 2H), 7.38 (d, *J* = 8.0 Hz, 2H), 7.19 (d, *J* = 7.7 Hz, 2H), 7.10 (d, *J* = 7.7 Hz, 2H), 6.49-6.30 (m, 2H), 5.82 (d, *J* = 6.4 Hz, 1H), 5.01 (s, 1H), 4.93-4.92 (m, 1H), 4.89 (s, 1H), 4.83 (s, 2H), 4.58-4.57 (m, 1H), 4.42-4.31 (m, 2H), 3.21 (dd, *J* = 13.9, 7.0 Hz, 1H), 2.99 (d, *J* = 6.7 Hz, 2H), 2.45 (d, *J* = 7.1 Hz, 2H), 2.09 (s, 3H), 2.01 (d, *J*= 6.8 Hz, 2H), 1.85 (dt, *J*= 13.3, 6.7 Hz, 1H), 1.60 (s, 2H), 1.47 (s, 4H), 1.38 (d, *J* = 6.8 Hz, 3H), 0.99 (s, 3H), 0.90 (d, *J* = 6.6 Hz, 6H). $^{13}$C NMR (126 MHz, $CDCl_3$) $\delta$ 194.56, 170.79, 166.38, 152.57, 150.20, 150.07, 148.49, 147.40, 142.14, 140.45, 140.09, 131.70, 130.66, 129.96, 129.30, 127.11, 125.95, 112.38, 111.02, 110.13, 109.28, 70.02, 66.20, 52.77, 45.13, 44.42, 41.93, 39.90, 38.79, 33.13, 30.29, 27.13, 24.90, 22.48 (d, *J* = 3.1 Hz), 21.08, 18.13, 16.69. HRMS (ESI) m/z ($[M+H]^+$) calcd for $C_{37}H_{49}O_2$: 525.3727. Found: 525.3722.

**Example 6: Preparation of compound 6**

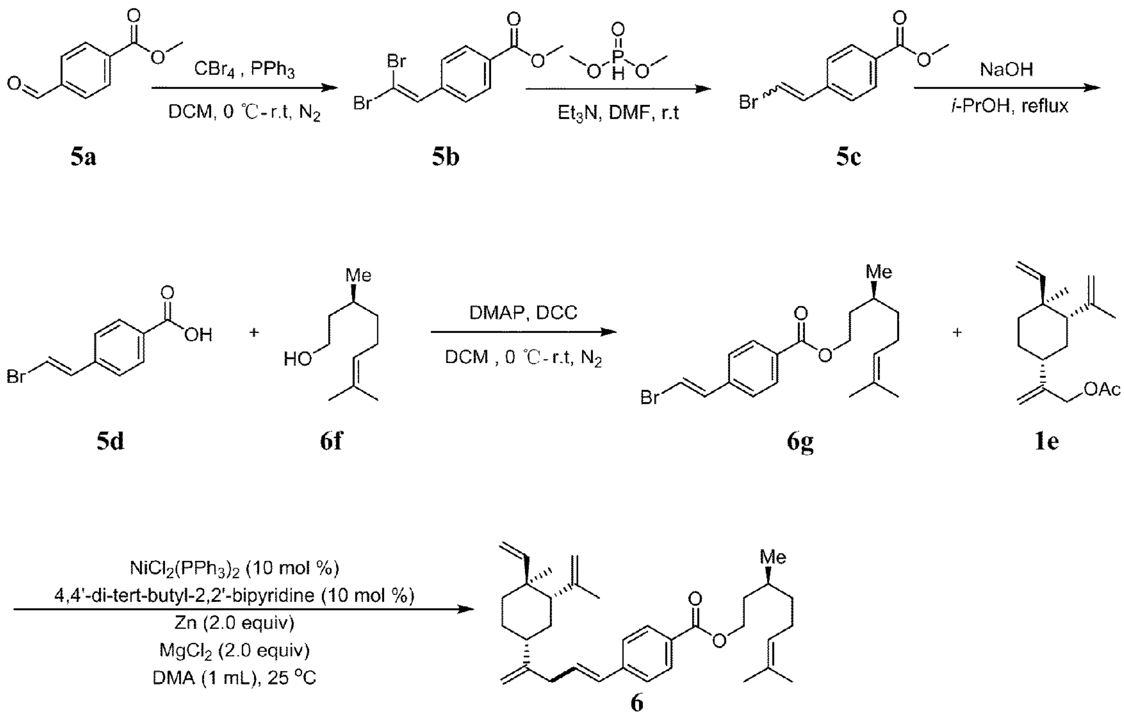


5a 5b 5c

5d 6f 6g 1e

6

Intermediate **6g**

Referring to the synthesis steps of the intermediate **5g** in Example 5, a colorless oily liquid 6g (267 mg, yield 82%) was obtained. $^{1}$H NMR (500 MHz, $CDCl_3$) $\delta$ 7.98 (d, $J$ = 8.3 Hz, 2H), 7.34 (d, $J$ = 8.3 Hz, 2H), 7.13 (d, $J$ = 14.0 Hz, 1H), 6.91 (d, $J$ = 14.0 Hz, 1H), 5.09 (t, $J$ = 7.1 Hz, 1H), 4.40-4.29 (m, 2H), 1.99 (dd, $J$ = 22.5, 7.6 Hz, 2H), 1.80 (dt, $J$ = 12.5, 6.3 Hz, 1H), 1.66 (s, 3H), 1.59 (s, 3H), 1.34-1.28 (m, 2H), 1.24 (dd, $J$= 15.0, 7.7 Hz, 2H), 0.96 (d, $J$= 6.6 Hz, 3H).

Compound **6**

Referring to the synthesis steps of the Compound 1 in Example 1, a colorless oily liquid **6** (63.7 mg, yield 87%) was obtained. $^{1}$H NMR (500 MHz, $CDCl_3$) $\delta$ 7.97 (d, $J$ = 7.9 Hz, 2H), 7.41 (d, $J$ = 8.0 Hz, 2H), 6.50-6.29 (m, 2H), 5.82 (dd, $J$ = 17.4, 10.8 Hz, 1H), 5.10 (dd, $J$ = 6.9, 5.9 Hz, 1H), 4.92 (s, 1H), 4.91 (s, 1H), 4.89 (d, $J$ = 2.6 Hz, 1H), 4.82 (d, $J$ = 5.1 Hz, 2H), 4.60 (s, 1H), 4.46-4.27 (m, 2H), 2.99 (d, $J$ = 6.8 Hz, 2H), 2.07-1.97 (m, 4H), 1.92 (d, $J$ = 10.1 Hz, 1H), 1.86-1.79 (m, 1H), 1.75 (d, $J$= 9.2 Hz, 1H), 1.71 (s, 3H), 1.68 (s, 3H), 1.61 (s, 3H), 1.56 (d, $J$ = 8.1 Hz, 1H), 1.51-1.44 (m, 3H), 1.31 (dd, $J$ = 17.7, 7.5 Hz, 4H), 1.01 (s, 3H), 0.97 (d, $J$ = 6.5 Hz, 3H). $^{13}$C NMR (126 MHz, $CDCl_3$) $\delta$ 166.64, 152.66, 150.27, 147.72, 142.14, 131.72, 131.48, 130.72, 130.00, 129.01 125.99, 124.71, 112.30, 110.08, 109.31, 63.53, 52.87, 44.47, 40.00 (d, $J$ = 10.8 Hz), 38.84, 37.13, 35.66, 35.06, 33.35, 29.72, 27.30, 25.84, 25.54, 24.93, 19.65, 17.80, 16.75. HRMS (ESI) m/z ([M+H]$^{+}$) calcd for $C_{34}H_{49}O_2$: 489.3727. Found: 489.3730.

**Example 7: Preparation of compound 7**

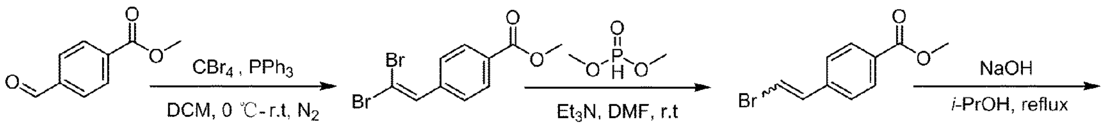


5a 5b 5c

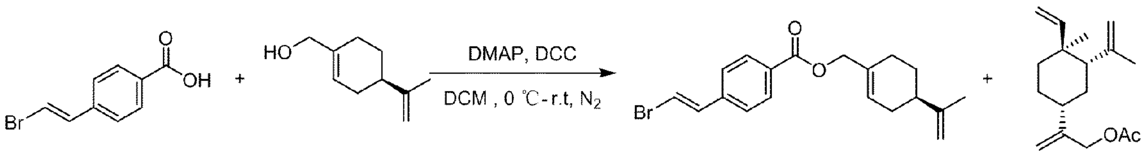


5d 7f 7g 1e

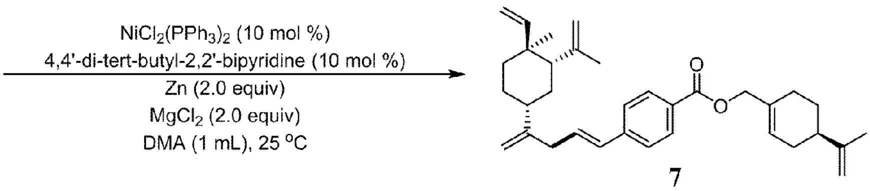


7

Intermediate **7g**

Referring to the synthesis steps of the intermediate **5g** in Example 5, a colorless oily liquid 7g (281 mg, yield 89%) was obtained. $^1$H NMR (500 MHz, $CDCl_3$) $\delta$ 8.00 (d, *J* = 8.2 Hz, 2H), 7.35 (d, *J* = 8.3 Hz, 2H), 7.13 (d, *J* = 14.0 Hz, 1H), 6.91 (d, *J* = 14.0 Hz, 1H), 5.84 (s, 1H), 4.73-4.70 (m, 3H), 2.17 (d, *J* = 3.6 Hz, 1H), 1.93-1.89 (m, 2H), 1.74 (s, 3H), 1.58-1.49 (m, 2H), 1.30 (d, *J* = 9.8 Hz, 2H).

Compound 7

Referring to the synthesis steps of the Compound 1 in Example 1, a colorless oily liquid 7 (59.5 mg, yield 82%) was obtained. $^1$H NMR (500 MHz, $CDCl_3$) $\delta$ 7.97 (d, *J* = 8.3 Hz, 2H), 7.40 (d, *J* = 8.3 Hz, 2H), 6.38 (dt, *J* = 15.8, 12.4 Hz, 2H), 5.82-5.80 (m, 2H), 5.00 (s, 1H), 4.91 (s, 1H), 4.88 (d, *J* = 2.1 Hz, 1H), 4.81 (s, 2H), 4.71 (dd, *J* = 8.9, 5.0 Hz, 4H), 4.57 (s, 1H), 2.98 (d, *J*= 6.7 Hz, 2H), 2.17 (d, *J*= 5.1 Hz, 3H), 2.08 (s, 3H), 2.01 (s, 2H), 1.90 (d, *J*= 10.0 Hz, 3H), 1.70-1.69 (m, 3H), 1.59 (s, 2H), 1.46 (d, *J* = 3.4 Hz, 4H), 1.29 (s, 1H), 1.00-0.99 (m, 3H). $^{13}$C NMR (126 MHz, $CDCl_3$) $\delta$ 194.56, 170.79, 154.90, 152.57, 150.06, 148.48, 147.40, 132.89, 131.73, 130.66, 130.02, 125.96, 125.59, 112.36, 111.01, 110.12, 108.90, 68.78, 66.20, 55.80, 52.76, 48.46, 45.71, 44.42, 41.92, 40.95, 39.89, 38.79, 36.51, 35.00, 33.12, 30.56. HRMS (ESI) m/z ([M+H]$^+$) calcd for $C_{34}H_{45}O_2$: 485.3414. Found: 485.3409.

**Example 8: Preparation of compound 8**

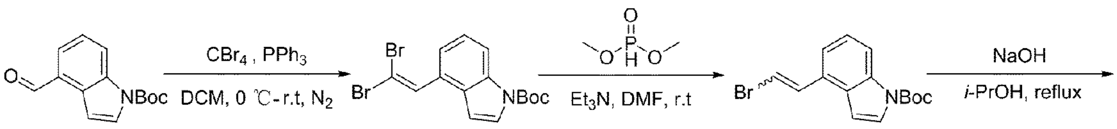


8a 8b 8c

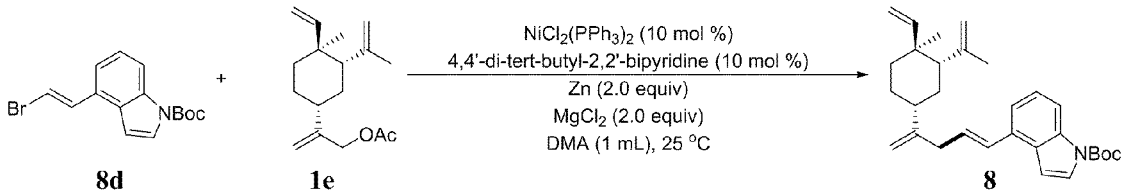


8d 1e 8

Compound **8**

Referring to the synthesis steps of the Compound **1** in Example 1, a colorless oily liquid **8** (50.1 mg, yield 75%) was obtained. $^1$H NMR (500 MHz, $CDCl_3$) $\delta$ 8.01 (d, *J* = 7.7 Hz, 1H), 7.59 (d, *J* = 3.6 Hz, 1H), 7.32 (d, *J* = 7.5 Hz, 1H), 7.24 (t, *J* = 3.9 Hz, 1H), 6.72 (d, *J* = 3.3 Hz, 2H), 6.32 (dt, *J* = 15.7, 7.1 Hz, 1H), 5.80 (dd, *J* = 17.4, 10.9 Hz, 1H), 4.93-4.77 (m, 5H), 4.58 (s, 1H), 3.02 (d, *J* = 7.0 Hz, 2H), 2.00 (dd, *J* = 12.0, 3.9 Hz, 2H), 1.70 (s, 3H), 1.65 (s, 12H), 1.46 (ddd, *J* = 9.0, 7.8, 2.6 Hz, 3H), 1.00 (s, 3H). $^{13}$C NMR (126 MHz, $CDCl_3$) $\delta$ 153.17, 150.37, 147.82, 135.65, 134.23, 130.42 (d, *J*= 8.5 Hz), 128.63, 125.92, 124.48, 119.37, 115.42, 114.66, 113.93, 112.29, 110.04, 109.06, 105.62, 83.83, 52.92, 44.45, 40.05 (d, *J* = 12.6 Hz), 39.20, 33.41, 29.85, 28.34, 27.36, 24.94, 16.78. HRMS (ESI) m/z ($[M-H]^-$) calcd for $C_{30}H_{38}NO_2$: 444.2908. Found: 444.2908.

**Example 9: Preparation of compound 9**

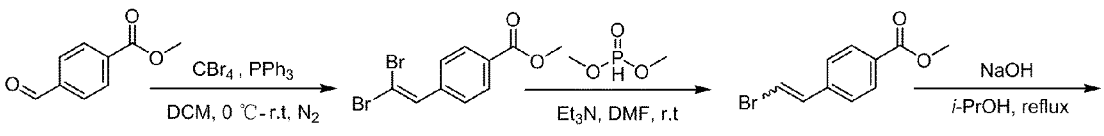


5a 5b 5c

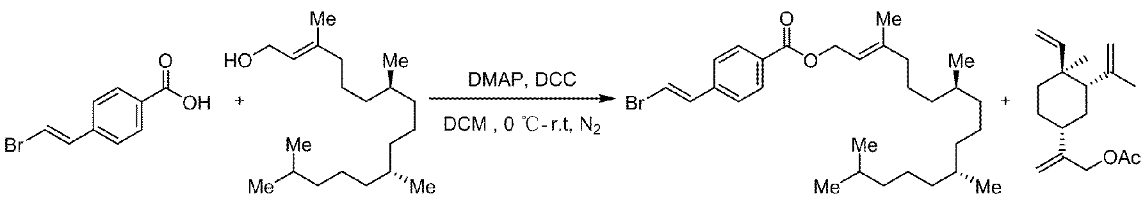


5d 9f 9g 1e

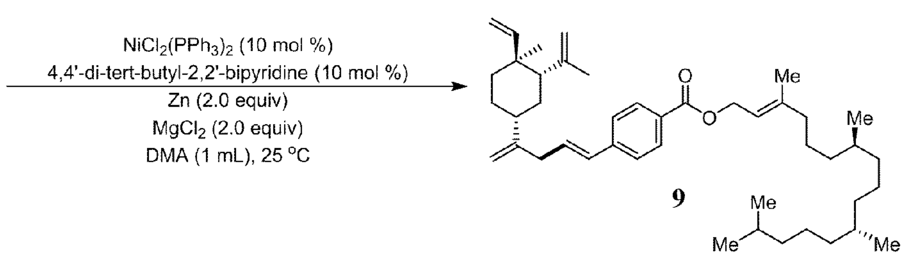


9

Intermediate **9g**

Referring to the synthesis steps of the intermediate **5g** in Example 5, a colorless oily liquid **9g** (416 mg, yield 69%) was obtained. $^1$H NMR (500 MHz, $CDCl_3$) $\delta$ 7.99 (d, *J* = 8.4 Hz, 2H), 7.34 (d, *J* = 8.3 Hz, 2H), 7.13 (d, *J* = 14.1 Hz, 1H), 6.90 (d, *J* = 14.0 Hz, 1H), 5.45 (t, *J* = 7.0 Hz, 1H), 4.83 (d, *J* = 7.0 Hz, 2H), 2.04 (d, *J* = 5.1 Hz, 2H), 1.75 (s, 3H), 1.51 (dd, *J* = 13.3, 6.7 Hz, 1H), 1.39-1.11 (m, 20H), 0.88-0.81 (m, 12H).

Compound **9**

Referring to the synthesis steps of the Compound **1** in Example 1, a colorless oily liquid **9** (73.6 mg, yield 78%) was obtained. $^1$H NMR (500 MHz, $CDCl_3$) $\delta$ 7.98 (d, *J* = 8.4 Hz, 2H), 7.40 (d, *J* = 8.4 Hz, 2H), 6.54-6.26 (m, 2H), 5.82 (dd, *J* = 17.3, 11.0 Hz, 1H), 5.46 (dd, *J* = 7.0, 6.0 Hz, 1H), 4.93-4.89 (m, 2H), 4.83 (dd, *J* = 5.9, 4.0 Hz, 3H), 4.60 (s, 1H), 3.20 (dt, *J* = 13.3, 5.0 Hz, 1H), 2.99 (d, *J* = 6.8 Hz, 2H), 2.07-1.98 (m, 5H), 1.95-1.89 (m, 2H), 1.75 (s, 3H), 1.71 (s, 3H), 1.63-1.57 (m, 5H), 1.48 (ddd, *J* = 13.1, 9.9, 5.1 Hz, 8H), 1.16-1.12 (m, 5H), 1.09-1.05 (m, 5H), 1.01 (s, 3H), 0.86 (d, *J* = 5.5 Hz, 12H). $^{13}$C NMR (126 MHz, $CDCl_3$) $\delta$ 166.66, 152.68, 150.29, 147.74, 142.87, 142.13, 131.69, 130.74, 130.07, 129.03, 125.98, 118.37, 112.31, 110.08, 109.32, 61.98, 55.89, 52.88, 44.47, 40.05, 39.79, 39.52, 38.85, 37.57, 37.51, 37.44, 36.77, 35.07, 33.36, 32.87 (d, *J* = 15.3 Hz), 29.85, 28.12, 27.30, 25.60, 25.19, 24.94, 24.61, 22.82 (d, *J* = 11.7 Hz), 19.88 (d, *J* = 3.7 Hz), 16.69 (d, *J* = 18.4 Hz), 14.27. HRMS (ESI) m/z ($[M+H]^+$) calcd for $C_{44}H_{69}O_2$: 629.5292. Found: 629.5291.

**Example 10: Preparation of compound 10**

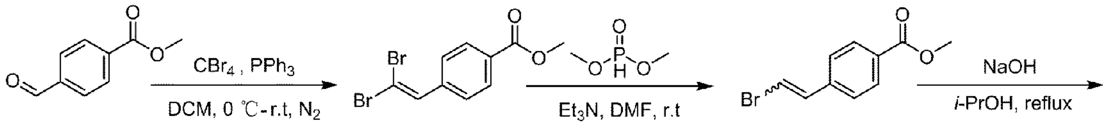


5a 5b 5c

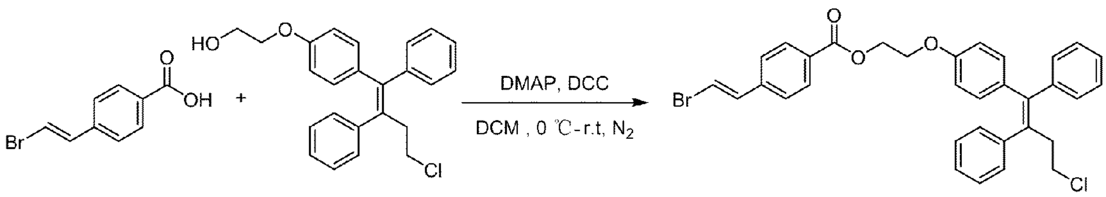


5d 10f 10g

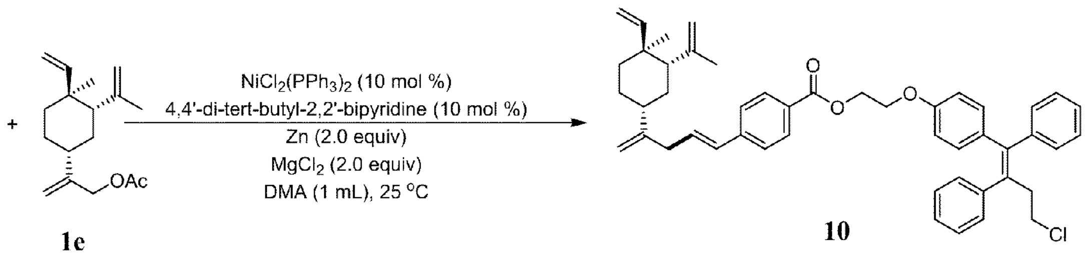


1e 10

Intermediate **10g**

Referring to the synthesis steps of the intermediate **5g** in Example 5, a white solid **10g** (1.07 g, yield 91%) was obtained. $^1$H NMR (500 MHz, $CDCl_3$) $\delta$ 7.97 (dd, *J* = 8.5, 1.6 Hz, 2H), 7.39-7.34 (m, 2H), 7.34-7.30 (m, 2H), 7.30-7.27 (m, 2H), 7.20 (dd, *J* = 8.1, 6.8 Hz, 2H), 7.16-7.10 (m, 4H), 6.91 (d, *J* = 14.1 Hz, 1H), 6.82-6.76 (m, 2H), 6.62-6.56 (m, 2H), 4.57 (dd, *J* = 5.5, 3.9 Hz, 2H), 4.16 (dd, *J* = 5.6, 3.9 Hz, 2H), 3.41 (t, *J* = 7.4 Hz, 2H), 2.92 (t, *J* = 7.5 Hz, 2H).

Compound **10**

Referring to the synthesis steps of the Compound 1 in Example 1, a colorless oily liquid **10** (86.4 mg, yield 81%) was obtained. $^1$H NMR (500 MHz, $CDCl_3$) $\delta$ 7.96 (d, *J* = 8.2 Hz, 2H), 7.42-7.36 (m, 4H), 7.31 (d, *J* = 6.2 Hz, 3H), 7.23-7.19 (m, 2H), 7.16 (d, *J* = 7.2 Hz, 3H), 6.82 (d, *J* = 8.7 Hz, 2H), 6.61 (d, *J* = 8.7 Hz, 2H), 6.51-6.31 (m, 2H), 5.86-5.80 (m, 1H), 5.11 (d, *J* = 13.8 Hz, 1H), 4.92 (s, 1H), 4.91 (s, 1H), 4.84 (d, *J* = 6.9 Hz, 2H), 4.61 (s, 1H), 4.59-4.56 (m, 2H), 4.20-4.14 (m, 2H), 3.43 (t, *J* = 7.4 Hz, 2H), 3.00 (d, *J* = 8.7 Hz, 2H), 2.95-2.91 (m, 2H), 2.05-2.00 (m, 2H), 1.73 (s, 3H), 1.62 (dd, *J* = 7.5, 3.3 Hz, 2H), 1.51-1.45 (m, 4H), 1.03 (s, 3H). $^{13}$C NMR (126 MHz, $CDCl_3$) $\delta$ 166.44, 156.86, 152.58, 150.24, 148.88, 147.68, 142.94, 142.41, 141.75, 141.03, 135.41 (d, *J* = 5.3 Hz), 131.93, 131.86, 130.62, 130.16, 129.63, 129.49, 128.46, 128.34, 127.07, 126.73, 125.98, 113.72, 113.13, 112.29, 110.07, 109.33, 79.52, 65.88, 63.33, 52.83, 44.43, 42.93, 41.65, 39.96 (d, *J* = 9.6 Hz), 38.75 (d, *J* = 14.8 Hz), 33.31, 29.81, 27.26, 24.92. HRMS (ESI) m/z ($[M+Na]^+$) calcd for $C_{48}H_{51}ClNaO_3$: 733.3419. Found: 733.3482.

**Example 11: Experiment of tumor cell proliferation inhibition**

**Evaluation of antitumor activity *in vitro***

1. Experimental equipment and reagents

1.1 Instrument

Biological safety cabinet (Shanghai Bio-Gene Biotechnology Co., Ltd.), constant-temperature carbon dioxide incubator (THERMO), enzyme-linked immunoassay analyzer (Spark), inverted microscope (Nikon), a set of pipette guns (eppendorf), and centrifuge (beckman coulter).

1.2 Reagents

McCoy'S 5A (Zhejiang Cienry Biotechnology Co., Ltd.), RPMI 1640 (Zhejiang Cienry Biotechnology Co., Ltd.), Fatal Bovine Serum (BI), PBS (Zhejiang Cienry Biotechnology Co., Ltd.), Trypsin (Zhejiang Cienry Biotechnology Co., Ltd.), DMSO (Coolaber), and CCK-8 (Coolaber).

1.3 Cell lines

Human colon cancer cells (HCT116), human lung cancer cells (A549).

2. Experimental methods

1) The test cells in a logarithmic growth phase were trypsinized and counted, inoculated in a 96-well culture plate at a concentration of $5\times10^4$/mL, 100 μL per well ($5\times10^3$ cells per well), and incubated at 37°C in a 5% $CO_2$ incubator for 24 h.
2) A drug to be tested was diluted to different concentrations with McCoy'S 5A or RPMI 1640 complete medium. The experimental group was replaced with a culture solution containing different concentrations of the tested samples, and the control group was replaced with a culture solution containing the same volume of solvent (DMSO). Three parallel wells were set up in each group, and the culture was continued for 48 h in a 5% $CO_2$ incubator at 37°C.

HCT116 cells used the McCoy'S 5A complete medium, and A549 cells used the RPMI 1640 complete medium.
3) 10 μL of a CCK-8 solution was added to each well, incubated at 37°C for 1 h to 4 h, and an absorbance (OD value) of each well at 490 nm was measured with a microplate reader.
4) A survival rate and an inhibition rate were calculated with the following formulas:

$$\text{Cell survival rate} = [(A_s - A_b)/(A_c - A_b)] \times 100\%$$

$$\text{Inhibition rate} = [(A_c - A_s)/(A_c - A_b)] \times 100\%$$

An S-type dose-survival curve was plotted using GraphPad Prism 7.0 software and a nonlinear regression model, and $IC_{50}$ values were calculated.

$A_s$: absorbance of the experimental well (medium containing cells, CCK-8, and the drug to be tested)

$A_c$: absorbance of control wells (medium containing cells, CCK-8, and solvent (DMSO))
$A_b$: absorbance of blank wells (medium without cells and drug to be tested, CCK-8)

3. Experimental results

The inhibitory effects of the target compound and the positive control drug β-elemene on the proliferation of two tumor cells were determined according to the above experimental method. The results were shown in Table 1.

Table 1 Effect of target compounds on tumor cell inhibition rate

| Compound No. | Anti-proliferative activity at 100 μM by concentration (%) | |
|---|---|---|
| | HCT116 | A549 |
| **4** | 99.8±0.08 | 97.5±0.4 |
| **6** | 21.7±0.8 | 14.4±0.5 |
| **10** | 13.5±0.3 | 40.4±0.6 |
| **β-elemene** | 0.6±0.5 | 10.6±0.5 |

100 μM of the compound acted on tumor cells for 48 h.

The results showed that Compound **4** showed an anti-tumor cell proliferation effect significantly stronger than that of the positive control β-elemene.

The compound 4 was selected to determine the $IC_{50}$ value according to the above experimental method. The results were shown in Table 2.

Table 2 $IC_{50}$ of target compounds on tumor cells

| Compound No. | $IC_{50}$ (μM) | |
|---|---|---|
| | HCT116 | A549 |
| **4** | 50.9±0.8 | 57.21±2.95 |
| **β-elemene** | 781.9±51 | 227.0±1.2 |

The compounds with different concentrations acted on tumor cells for 48 h.

The results showed that Compound **4** exhibited significantly stronger cell proliferation-inhibiting activity than the positive control β-elemene.

In addition, it should be understood that various changes or modifications may be made to the present disclosure by those skilled in the art after reading the above teaching content of the present disclosure, and these equivalent forms also fall within the scope defined by the appended claims of the present disclosure.

## Claims

1. A vinylation coupled derivative of β-elemene, or an optical isomer, a racemate, a single enantiomer, and a diastereomer thereof, or a pharmaceutically acceptable salt, a prodrug, a deuterated derivative, a hydrate, and a solvate thereof, wherein the vinylation coupled derivative of β-elemene has a structure shown in formula (I):

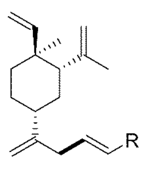

Formula (I);

and
R is selected from the group consisting of aryl, heteroaryl, and alkyl in formula (I).

**2.** The vinylation coupled derivative of β-elemene, or an optical isomer, a racemate, a single enantiomer, and a diastereomer thereof, or a pharmaceutically acceptable salt, a prodrug, a deuterated derivative, a hydrate, and a solvate thereof according to claim 1, wherein R in the structure shown in Formula (I) of the vinylation coupled derivative of β-elemene is any one independently selected from the group consisting of the following structural fragments:

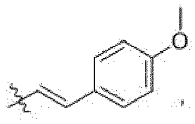,
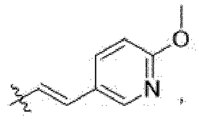,
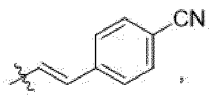,
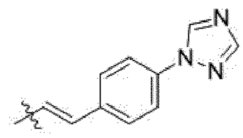,
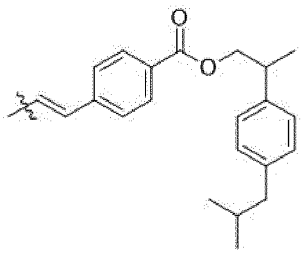,
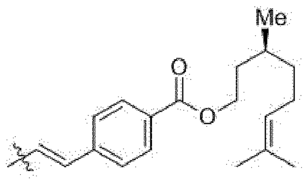,
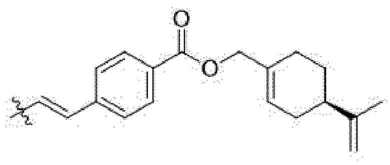,
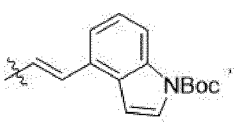,
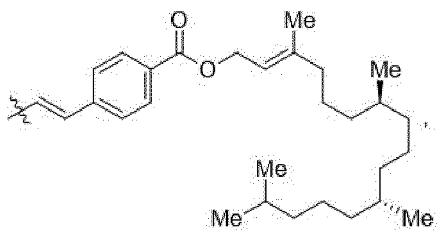,
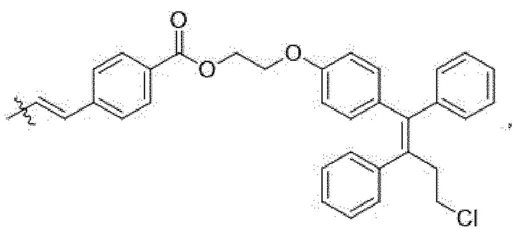,
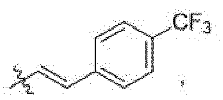,
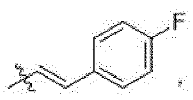,
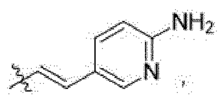,
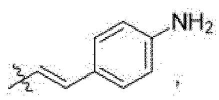,
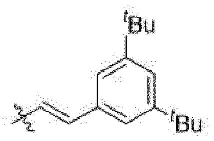,
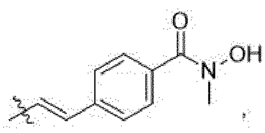,
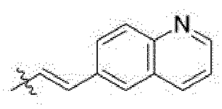,
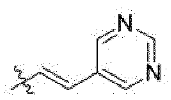,
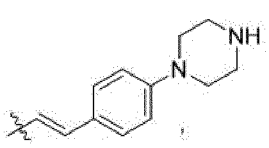,
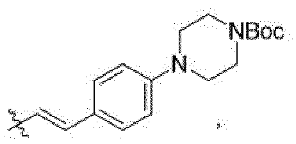,
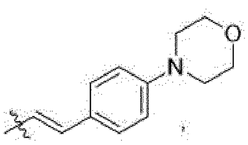,
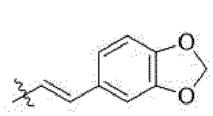.

**3.** The vinylation coupled derivative of β-elemene, or an optical isomer, a racemate, a single enantiomer, and a diastereomer thereof, or a pharmaceutically acceptable salt, a prodrug, a deuterated derivative, a hydrate, and a solvate thereof according to claim 1, wherein the vinylation coupled derivative of β-elemene has a structure shown in any one of Compounds 1 to 22:

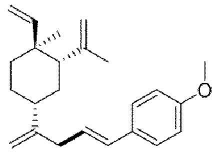

1

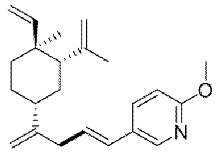

2

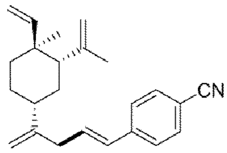

3

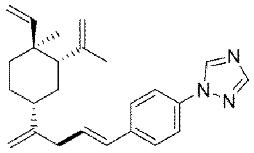

4

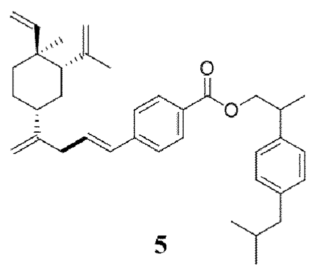

5

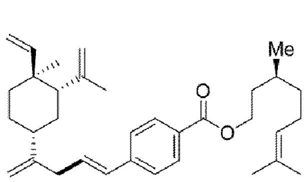

6

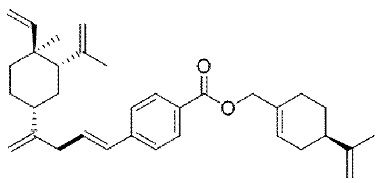

7

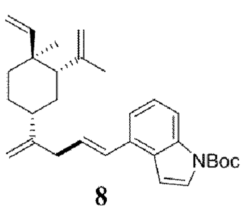

8

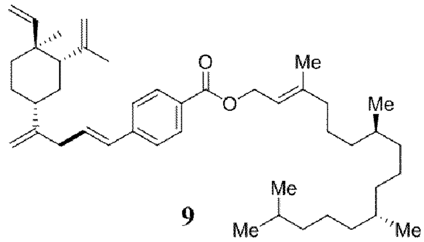

9

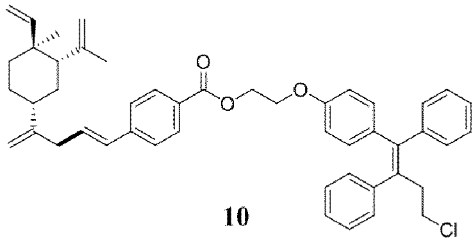

10

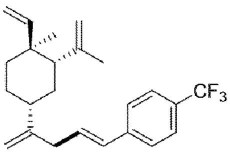

11

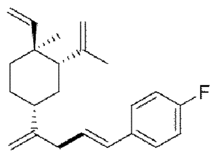

12

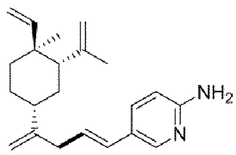

13

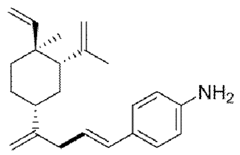

14

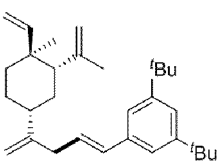

15

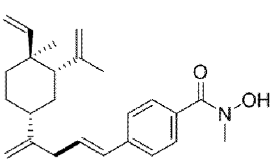

16

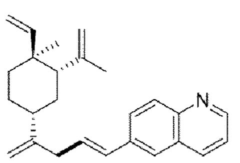

17

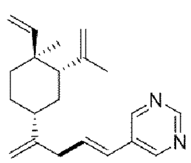

18

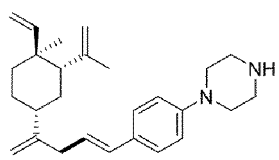

19

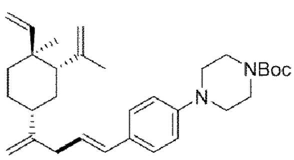

20

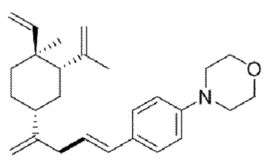

21

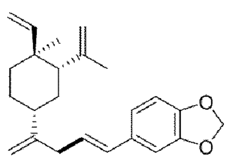

22

.

**4.** A preparation method of the vinylation coupled derivative of β-elemene according to any one of claims 1 to 3, wherein the preparation method has the following synthetic route:

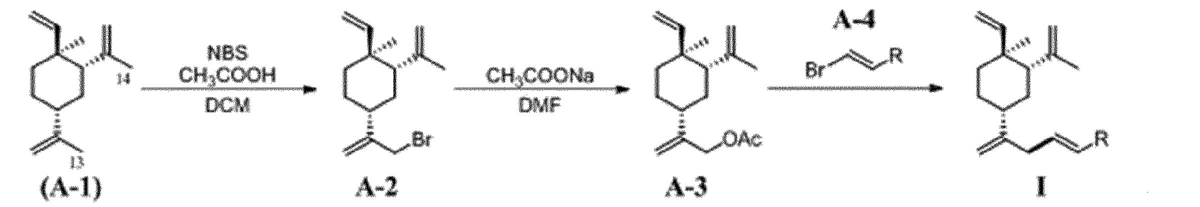


(A-1) A-2 A-3 I

β-elemene

R = aryl, heteroaryl, and alkyl

; and

the preparation method comprises the following steps:

(1) subjecting β-elemene A-1 to monobromine substitution at an allyl position to obtain an intermediate A-2;
(2) subjecting the intermediate A-2 to selective nucleophilic substitution to obtain an intermediate A-3; and
(3) allowing the intermediate A-3 and alkenyl bromide A-4 to have vinylation coupling to obtain the vinylation coupled derivative of β-elemene having a structure shown in Formula (I); wherein

R is any one independently selected from the group consisting of the following structural fragments:

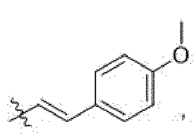, 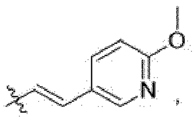, 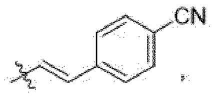, 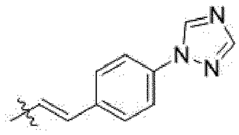,

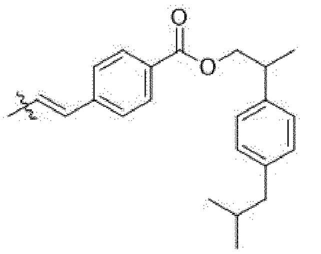, 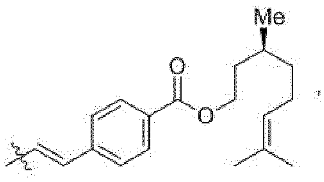, 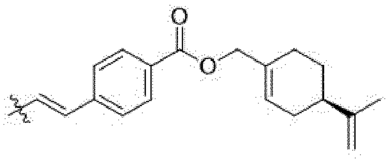,

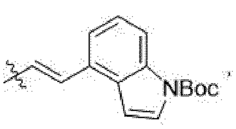, 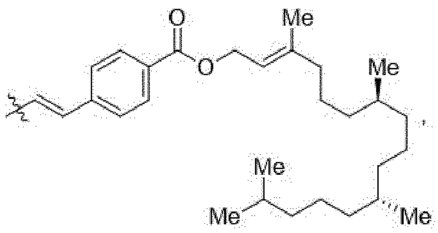, 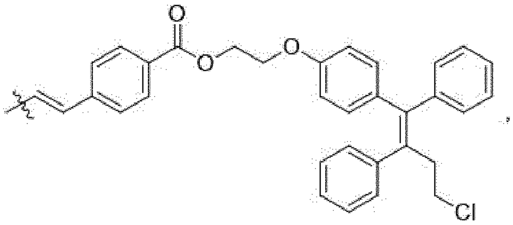,

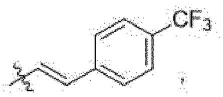, 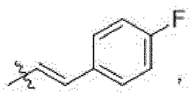, 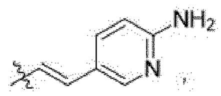, 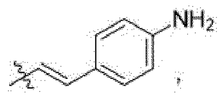,

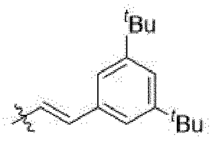, 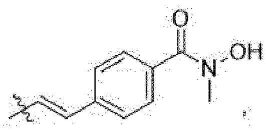, 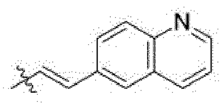, 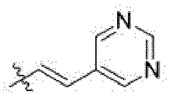;

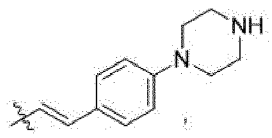, 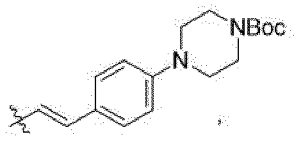, 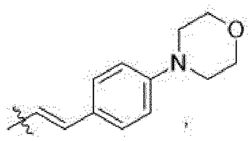, 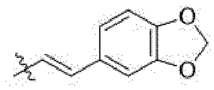.

**5.** Use of the vinylation coupled derivative of β-elemene, or an optical isomer, a racemate, a single enantiomer, and a diastereomer thereof, or a pharmaceutically acceptable salt, a prodrug, a deuterated derivative, a hydrate, and a solvate thereof according to any one of claims 1 to 3 in preparation of an antitumor drug.

**6.** The use according to claim 5, wherein the antitumor drug is used for treating colon cancer, lung cancer, liver cancer, gastric cancer, prostate cancer, ovarian cancer, breast cancer, or glioma.

**7.** The use according to claim 5, wherein the vinylation coupled derivative of β-elemene, or an optical isomer, a racemate, a single enantiomer, and a possible diastereomer thereof, or a pharmaceutically acceptable salt, a prodrug, a deuterated derivative, a hydrate, and a solvate thereof according to any one of claims 1 to 3 is applied by oral administration, intratumoral administration, rectal administration, parenteral administration, and topical administration.

**8.** The use according to claim 7, wherein a solid dosage form for the oral administration comprises a capsule, a tablet, a pill, a powder, and a granule;
in the solid dosage form, the vinylation coupled derivative of β-elemene, or an optical isomer, a racemate, a single enantiomer, and a possible diastereomer thereof, or a pharmaceutically acceptable salt, a prodrug, a deuterated derivative, a hydrate, and a solvate thereof according to any one of claims 1 to 3 is mixed with at least one inert excipient or carrier; the inert excipient or carrier is selected from the group consisting of sodium citrate and dicalcium phosphate; alternatively, the vinylation coupled derivative of β-elemene, or an optical isomer, a racemate, a single enantiomer, and a possible diastereomer thereof, or a pharmaceutically acceptable salt, a prodrug, a deuterated derivative, a hydrate, and a solvate thereof is mixed with a filler or a solubilizer, a binder, a humectant, a disintegrant, a retarding solvent, an absorption accelerator, a wetting agent, an adsorbent, and a lubricant; the filler or the solubilizer is selected from the group consisting of starch, lactose, sucrose, glucose, mannitol, and silicic acid; the binder is selected from the group consisting of hydroxymethylcellulose, an alginate, gelatin, polyvinylpyrrolidone, sucrose, and arabic gum; the humectant is glycerin; the disintegrant is selected from the group consisting of agar, calcium carbonate, potato starch or tapioca starch, alginic acid, a complex silicate, and sodium carbonate; the retarding solvent is paraffin; the absorption accelerator is a quaternary ammonium compound; the wetting agent is selected from the group consisting of cetyl alcohol and glyceryl monostearate; the adsorbent is kaolin; and the lubricant is selected from the group consisting of talc, calcium stearate, magnesium stearate, solid polyethylene glycol, and sodium lauryl sulfate and a mixture thereof.

**9.** The use according to claim 5, wherein a daily dosage is 1 mg to 5,000 mg for a person weighing 60 kg.

**10.** An antitumor drug, comprising a safe and effective dosage of the vinylation coupled derivative of β-elemene, or an optical isomer, a racemate, a single enantiomer, and a possible diastereomer thereof, or a pharmaceutically acceptable salt, a prodrug, a deuterated derivative, a hydrate, and a solvate thereof according to any one of claims 1 to 3.

**11.** The antitumor drug according to claim 10, further comprising a pharmaceutically acceptable excipient or carrier.

**12.** The antitumor drug according to claim 10 or 11, wherein the antitumor drug is used for treating colon cancer and lung cancer.

**13.** The antitumor drug according to claim 10, wherein the antitumor drug carries the vinylation coupled derivative of β-elemene, or an optical isomer, a racemate, a single enantiomer, and a possible diastereomer thereof, or a pharmaceutically acceptable salt, a prodrug, a deuterated derivative, a hydrate, and a solvate thereof according to any one of claims 1 to 3 at 1 mg/dose to 2,000 mg/dose; and one dose refers to one capsule or one tablet.

**14.** The antitumor drug according to claim 10, wherein the pharmaceutically acceptable carrier comprises cellulose and a derivative thereof, gelatin, talc, a solid lubricant, calcium sulfate, vegetable oil, a polyol, an emulsifier, a wetting agent, a coloring agent, a flavoring agent, a stabilizer, an antioxidant, a preservative, and pyrogen-free water.

**15.** The antitumor drug according to claim 10, wherein a tablet, a dragee, a capsule, a pill, and a granule each are prepared from a coating and a shell material.

**INTERNATIONAL SEARCH REPORT**

International application No.
**PCT/CN2022/132420**

**A. CLASSIFICATION OF SUBJECT MATTER**

C07C 43/215(2006.01)i; C07C 41/30(2006.01)i; C07C 69/767(2006.01)i; C07C 67/343(2006.01)i; C07C 255/50(2006.01)i; C07C 253/30(2006.01)i; C07C 69/76(2006.01)i; C07C 22/08(2006.01)i; C07C 17/263(2006.01)i; C07C 25/24(2006.01)i; C07C 211/45(2006.01)i; C07C 209/68(2006.01)i; C07C 13/28(2006.01)i; C07C 259/10(2006.01)i; C07D 213/64(2006.01)i; C07D 249/08(2006.01)i; C07D 209/08(2006.01)i; C07D 213/73(2006.01)i; C07D 215/06(2006.01)i; C07D 239/26(2006.01)i; C07D 295/033(2006.01)i; C07D 295/205(2006.01)i; C07D 317/50(2006.01)i; C07B 37/00(2006.01)i; A61K 31/085(2006.01)i; A61K 31/277(2006.01)i; A61K 31/235(2006.01)i; A61K 31/03(2006.01)i; A61K 31/136(2006.01)i; A61K 31/015(2006.01)i; A61K 31/166(2006.01)i; A61K 31/44(2006.01)i; A61K 31/4196(2006.01)i; A61K 31/404(2006.01)i; A61K 31/47(2006.01)i; A61K 31/505(2006.01)i; A61K 31/495(2006.01)i; A61K 31/5375(2006.01)i; A61K 31/36(2006.01)i; A61P 35/00(2006.01)i

According to International Patent Classification (IPC) or to both national classification and IPC

**B. FIELDS SEARCHED**

Minimum documentation searched (classification system followed by classification symbols)

C07C; C07D; C07B; A61K; A61P

Documentation searched other than minimum documentation to the extent that such documents are included in the fields searched

Electronic data base consulted during the international search (name of data base and, where practicable, search terms used)

CNABS; CNTXT; CNKI; VEN; WOTXT; EPTXT; USTXT; STN: 杭州师范大学, 谢恬, 叶杨, 徐冰, 叶向阳, 榄香烯, 衍生物, 乙烯, 偶联, 肿瘤, 癌, 增殖, elemene, derivative, vinyl+, coupling, cancer, carcinoma, antiproliferation, anti-proliferative, 结构式, structural formula

**C. DOCUMENTS CONSIDERED TO BE RELEVANT**

| Category* | Citation of document, with indication, where appropriate, of the relevant passages | Relevant to claim No. |
|---|---|---|
| PX | CN 114524716 A (HANGZHOU NORMAL UNIVERSITY) 24 May 2022 (2022-05-24)<br>claims 1-8, and description, paragraphs [0006]-[0038] | 1-15 |
| PX | YE, Yang et al. "Nickel-Catalyzed Cross-Electrophile Allylation of Vinyl Bromides and the Modification of Anti-Tumour Natural Medicine β-elemene"<br>*Chemical Science,* Vol. 13, No. 23, 12 May 2022 (2022-05-12),<br>ISSN: 1742-2183,<br>pages 6959-6966 | 1-15 |
| A | EP 3034610 A1 (KITASATO INSTITUTE et al.) 22 June 2016 (2016-06-22)<br>description, paragrapahs [0024], [0126], and [0127] | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 January 2023** | **18 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/132420**

**C. DOCUMENTS CONSIDERED TO BE RELEVANT**

| Category* | Citation of document, with indication, where appropriate, of the relevant passages | Relevant to claim No. |
|---|---|---|
| A | CN 110683932 A (HANGZHOU NORMAL UNIVERSITY) 14 January 2020 (2020-01-14)<br>entire document | 1-15 |
| A | CN 1850779 A (SHENYANG PHARMACEUTICAL UNIVERSITY) 25 October 2006 (2006-10-25)<br>entire document | 1-15 |
| A | BAI, Renren et al. "Rational Design, Synthesis and Biological Evaluation of Novel Derivatives Based on In Vivo Metabolism of Natural Product β-elemene"<br>*Letters in Drug Design & Discovery,* Vol. 15, No. 8, 31 August 2018 (2018-08-31), ISSN: 1875-628X,<br>pages 905-912 | 1-15 |

Form PCT/ISA/210 (second sheet) (January 2015)

**INTERNATIONAL SEARCH REPORT**
**Information on patent family members**

International application No.
**PCT/CN2022/132420**

| Patent document cited in search report | | | Publication date (day/month/year) | Patent family member(s) | | | Publication date (day/month/year) |
|---|---|---|---|---|---|---|---|
| CN | 114524716 | A | 24 May 2022 | | None | | |
| EP | 3034610 | A1 | 22 June 2016 | JP | WO2015022798 | A1 | 02 March 2017 |
| | | | | WO | 2015022798 | A1 | 19 February 2015 |
| CN | 110683932 | A | 14 January 2020 | CN | 110683932 | B | 27 May 2022 |
| CN | 1850779 | A | 25 October 2006 | CN | 1850779 | B | 29 August 2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202210185780
- CN 110683932 A